# EUROPEAN PATENT SPECIFICATION

(11) **EP 1 331 879 B1**
(45) Date of publication and mention of the grant of the patent: **03.09.2008**
(21) Application number: 01981706.3
(22) Date of filing: 17.10.2001
(51) Int. Cl.: A61B 5/05

(54) **NERVE STIMULATOR NEEDLE GUIDANCE SYSTEM**
NADELFÜHRUNGSSYSTEM ZUR NERVENSTIMULATION
SYSTEME DE GUIDAGE D'AIGUILLE NEUROSTIMULATRICE

(30) Priority: 17.10.2000 US 241141 P; 01.02.2001 US 773123; 29.05.2001 US 293612 P
(43) Date of publication of application: 06.08.2003
(73) Proprietor: Urmey, William F., Larchmont, NY 10538 (US)
(72) Inventor: Urmey, William F., Larchmont, NY 10538 (US)
(74) Representative: Murgatroyd, Susan Elizabeth
(86) International application number: PCT/US2001/032412
(87) International publication number: WO 2002/032289

(56) References cited:
- EP-A- 0 759 307
- GB-A- 2 115 700
- US-A- 5 853 373
- US-A- 5 885 219

## Description

### BACKGROUND OF THE INVENTION

### Field of the Invention

This invention relates generally to regional anesthesia and more particularly to an apparatus for nerve stimulator needle guidance.

### Antecedents of the Invention

Often, regional anesthesia has been found to be preferable to general anesthesia because of increased safety, the availability of postoperative pain control and decreased anesthetic costs.

Regional anesthesia delivery techniques strove to optimize administration of a local anesthetic in close proximity to a target or nerve plexus so as to establish a neural blockade.

A prevalent technique employed the use of nerve stimulators electrically coupled to a nerve stimulator needle. Such method was premised upon the phenomenon that an electrical pulse is capable of stimulating a motor nerve fiber to contract an innervated muscle or cause paraesthesia, in the case of sensory nerve stimulation.

The initial placement of the needle was dependent upon anatomic landmarks. Since anatomic landmarks varied from patient to patient, they constituted only an approximate starting zone or region to guide needle insertion. Successful administration was often dependent upon the skill and experience of the anesthesiologist. Multiple needle passes were required when the initial needle placement was not directly registered over the target nerve or nerve plexus or when the angle of introduction was anatomically incorrect.

Peripheral nerves have been stimulated with cutaneous electrodes at appropriate landmarks utilizing a coupling gel for monitoring the degree of neuromuscular blockade during general anaesthesia when neuromuscular relaxing or paralyzing drugs were intravenously administered.

US patent 5853373 discloses a bipolar needle with current flowing through a needle cannula and return current flowing through a conductive layer positioned on the outer surface of a thin walled plastic tube which surrounds the needle cannula. The plastic tube is fixed to the needle cannula by a non-conductive epoxy or other adhesive. The needle cannula cannot move relative to the plastic tube.

GB patent application 2115700A discloses an electrode assembly which includes a non-penetrating, spring loaded, skin contact cutaneous electrode. An outer electrode is grasped by the user's hand so that current flows from the spring loaded contact skin electrode, through the user's body and hand to the outer electrode.

US patent 5885219 discloses a combined ultrasonic (blood vessel locating) and nerve stimulation device which is carried with the bore of an administration needle. The device includes a blunt tip or end having a nerve stimulator electrode and ultrasonic crystal.

### BRIEF SUMMARY OF THE INVENTION

A nerve stimulator needle guidance system comprising an electrode for locating a cutaneous or subcutaneous point in proximity with a target nerve or nerve plexus, the electrode having a conductive tip and a bore extending through the electrode and the tip, characterised by a the tip having a smooth surface and nerve stimulator needle received within and axially moveable within the bore.

In one embodiment a cutaneous guiding electrode is coupled to a nerve stimulator to locate a target nerve registration point while depressing the patient's dermal layers. Thereafter a nerve stimulator needle, coupled to the nerve stimulator, is introduced into the patient through the bore at the located registration point.

Conventional nerve stimulation techniques are employed to properly position the needle tip adjacent to the target nerve for the administration of anaesthesia or other treatment.

The cutaneous electrode bore serves as a guide for the initial placement of the needle at the registration point and for guidance of the needle travel path toward the target nerve. Maintaining the dermal layers in a depressed or indented state by continued pressure on the cutaneous electrode serves to fix the location of the target nerve.

In an alternate embodiment, a cylindrical subcutaneous guiding electrode includes a bore which extends through a blunt tip. The subcutaneous electrode is introduced through the skin utilizing a needle carried in the bore and projecting beyond the tip.

After the guiding electrode tip reaches a desired depth beneath the skin, the needle is withdrawn from the tip and the electrode is manipulated in a sweep pattern while nerve stimulation current is applied to the tip to locate the target nerve.

After the target nerve is located, the nerve stimulation current through the guiding electrode is terminated. The position of the guiding electrode is fixed and a nerve stimulator needle is advanced through the bore and beyond the electrode tip, while applying a lower level of current through the needle. When appropriate muscle contractions are elicited at low current levels, the nerve stimulator needle has been properly positioned for administration of anesthesia through the nerve stimulator needle.

From the foregoing compendium, it will be appreciated that it is an aspect of the present invention to provide a nerve stimulator needle guidance system of the general character described which is not subject to the disadvantages of the antecedents of the invention.

A consideration of the present invention to provide a nerve stimulator needle guidance system of the general character described which increases the likelihood of the successful administration of regional anesthesia with a single needle pass.

Other aspects, features and considerations of the present invention in part will be obvious and in part will be pointed out hereinafter.

### BRIEF DESCRIPTION OF THE DRAWINGS

In the accompanying drawings in which are shown some of the various possible exemplary embodiments of the invention:

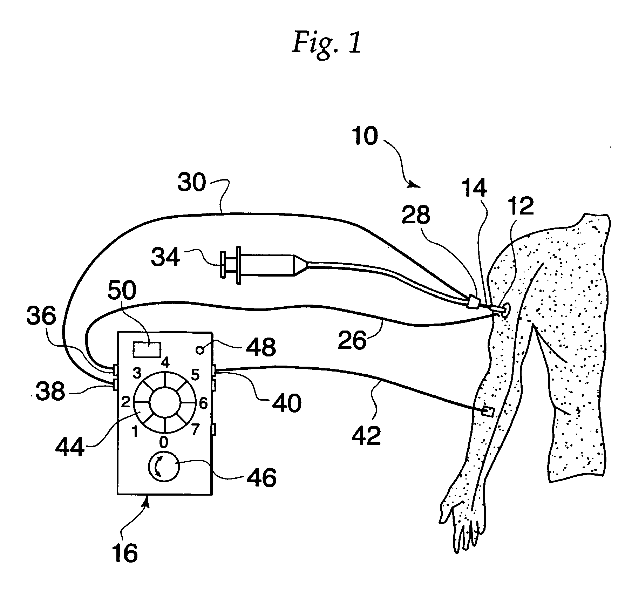

FIG. 1 is a schematized illustration of a nerve stimulator needle guidance system of the invention,

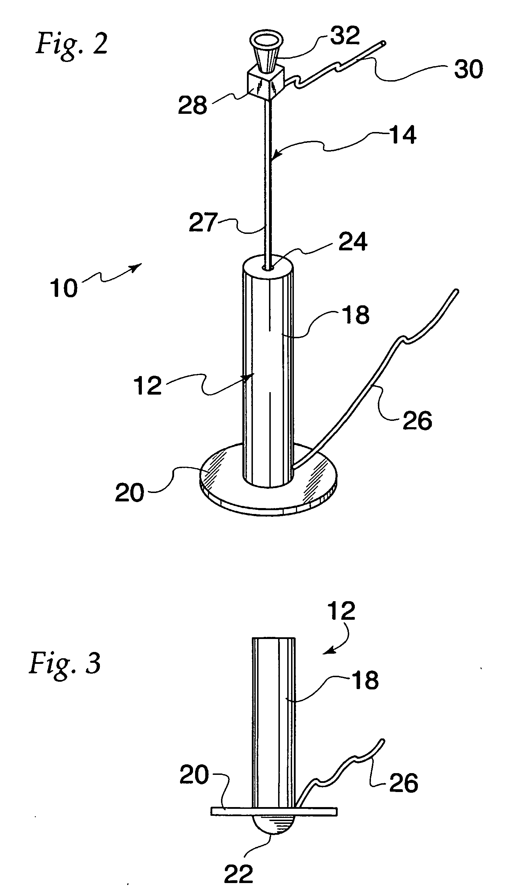

FIG. 2 is a perspective illustration of the nerve stimulator needle guidance system,
FIG. 3 is a front elevational view of the cutaneous electrode,
FIG. 4 is a sectional view through the nerve stimulator needle guidance system,
FIG. 5 is a perspective illustration of a further embodiment of the invention,
FIG. 6 is a perspective illustration of another embodiment of the invention,
FIG. 7 is an enlarged scale longitudinal sectional view through a subcutaneous guiding electrode illustrated in FIG. 6 with portions deleted for clarity,
FIG. 8 is a longitudinal sectional view through the guiding electrode and showing a nerve stimulator needle within a bore of the electrode,
FIG. 9 is a longitudinal sectional view through the subcutaneous guiding electrode after introduction beneath the patient's skin and showing anatomical components, not to scale,
FIG. 10 is a longitudinal sectional view similar to that of FIG. 9 and illustrating the process of locating a target nerve,
FIG. 11 is a longitudinal sectional view similar to FIG. 9 and FIG. 10, and
FIG. 12 is a longitudinal sectional view through further alternate embodiment of the invention, similar to the embodiment of FIG. 6, wherein a sensor is employed to automatically switch the output of a nerve stimulator.

### DESCRIPTION OF THE PREFERRED EMBODIMENTS

With reference now in detail to the drawings, the reference numeral 10 denotes a nerve stimulator needle guidance system in accordance with the invention. The system includes a cutaneous electrode 12 which is employed to locate a target nerve registration point on a patient's skin and a nerve stimulator needle electrode 14 which is introduced into the patient at the located registration point. Both electrodes are coupled to a nerve stimulator 16.

The cutaneous electrode 12 comprises a non-conductive body 18, depicted in the figures as having a cylindrical shape. A transverse flange 20 projects laterally from the lower end of the body 18 and a generally hemispherical electrically conductive tip 22 is positioned below the flange 20 and in registration with the body 18. A bore 24 extends coaxially through both the body and the conductive tip and an electrical lead 26 couples the conductive tip to the nerve stimulator 16.

The nerve stimulator needle 14 is of generally conventional configuration and includes a needle shaft 27 which may be coated with a nonconductive material, except for the tip portion thereof. At one end of the shaft 27 is a connector block 28 from which an electrical lead 30 extends to electrically couple the nerve stimulator needle 14 to the nerve stimulator 16. A Luer fitting 32 may be mounted to the connector block to connect the needle bore to a conventional syringe 34.

The nerve stimulator 16 includes known pulse generating and adjustable current control circuits such as those employed in the DIGISTIM III nerve stimulator sold by Neuro Technology Inc. of Houston, TX, or those disclosed in U.S. Patent 4,515,168.

Multiple base current setting levels and outputs are provided, however. One output terminal 36 is provided for the cutaneous electrode 12 and another output terminal 38 is provided for the nerve stimulator needle 14, with the respective electrical leads 26, 30 being coupled to their associated terminals.

The nerve stimulator 16 also includes a ground terminal 40 to which is coupled a ground output lead 42, the end of which is electrically coupled to the patient in a conventional manner.

A base level output current setting control 44 is operable to adjust to the optimal initial or base level currents and current ratios through the terminals 36, 38 suitable for employment in conjunction with a specified target nerve or nerve plexus.

For each of the various target nerves, e.g. femoral nerve, popliteal fossa approach to sciatic nerve, mid-humeral approach to median nerve, ulnar nerve, sciatic nerve, interscalene brachial plexus approach to C5 and C6 nerve roots, etc., optimal initial or base level current flow through the cutaneous electrode terminal 36 and the stimulator needle terminal 38 are provided.

The cutaneous electrode current flow may be within an overall range of 2 - 10 mA, for example, and nerve stimulator needle current flow may be within an overall range of .3 - 3.0 mA, for example. By way of further example, minimum cutaneous electrical current for location of the femoral nerve may be achieved at 5 mA while the minimum stimulator needle current flow may be at .5 mA.

The nerve stimulator 16 also includes a variable current adjustment control 46 and an output selector switch 48 for selecting either the cutaneous electrode terminal 36 or the nerve stimulator needle terminal 38. Also provided is a display 50 for quantitatively indicating current flow through the active terminal or other data.

It should be understood that the nerve stimulator needle guidance system 10 may be utilized with a conventional nerve stimulator having, for example, a single output and with operating room personnel disconnecting the cutaneous electrode lead 26 and connecting the nerve stimulator needle lead 30 after the target nerve has been cutaneously located. The output will simultaneously be adjusted to a reduced level upon introduction of the nerve stimulator needle into the patient.

It is also possible to practice the invention utilizing two separate nerve stimulators, one coupled to the cutaneous electrode and the other coupled to the nerve stimulator needle.

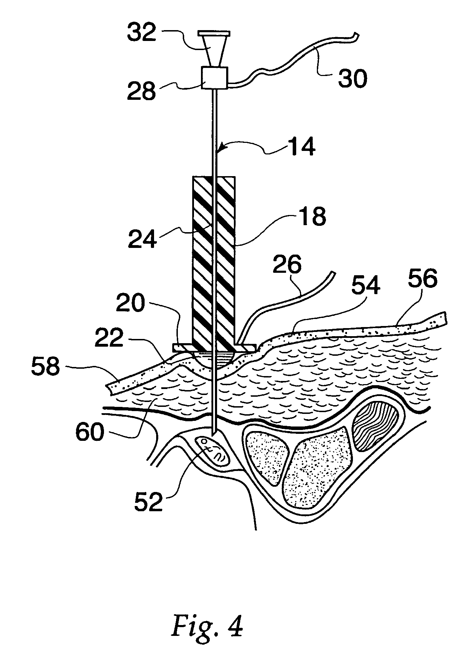

FIG. 4 illustrates use of the present invention in the administration of a nerve block to a femoral nerve 52. Initially, as under conventional practice, the patient receives prescribed amounts of intravenous sedatives in accordance with conventional routines for nerve location utilizing nerve stimulators.

A sterile coupling gel 54 may be applied over the patient's skin 56 in the appropriate anatomic region prelocated by landmarks. Thereafter, the cutaneous electrode 12, coupled to the nerve stimulator 16 through the lead 26, is forced against the skin 56 and underlying dermis 58, compressing subcutaneous fat 60 such that the flange 20 is essentially flush with the skin. The flange 20 may be fabricated of a transparent thermoplastic for ease in assuring that the proper tissue depression by the conductive tip 22 has been achieved.

The nerve stimulator output, with an initial or base current level set by the control 44, is moved across the skin to noninvasively elicit responses. By reducing current flow at the control 46, the cutaneous electrode 12 locates a point on the skin 56 which is in registration with the target femoral nerve 52. The registration point is found when it is determined that the appropriate muscle contractions have been elicited with minimum current, that is, maximal motor contractions have been encountered at a minimum current level.

The position of the cutaneous electrode 12 is held fast, i.e., is not moved laterally nor is the compressive force released and current flow through the cutaneous electrode 12 is terminated. The shaft 27 of the nerve stimulator needle 14 is then urged through the bore 24 and into the patient while being electrically coupled through the electrical lead 30 to the appropriate output terminal 38 of the nerve stimulator 16.

Utilizing conventional nerve stimulator techniques, the needle 14 is advanced through the bore while current flow is reduced through adjustment at the control 46 until stimulation of the femoral nerve is achieved utilizing a reduced current level associated with the prescribed distance between the tip of the needle shaft 27 and the target femoral nerve 52. Maintenance of the compressive force on the cutaneous electrode 18 serves to fix the position of the target nerve while the needle is being advanced.

Thereafter, a portion of the anesthetic dose is administered through the needle to terminate response to the stimulation current. If the response terminates with the initial administration, the needle is deemed to be properly positioned for the requisite procedure.

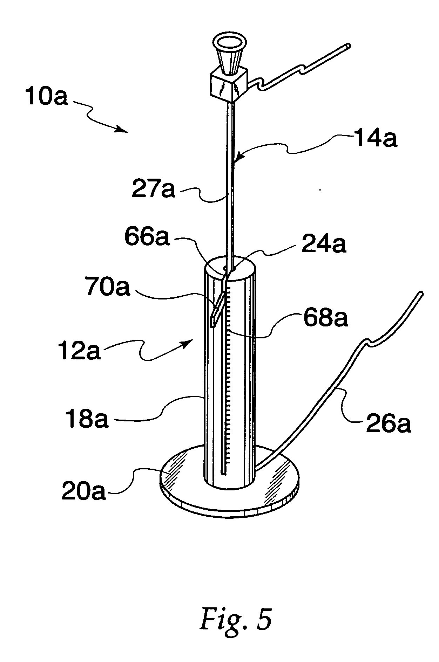

With reference now to FIG. 5, wherein an alternate embodiment of the invention is illustrated, like numerals have been employed to denote like components of the previous embodiment, however, bearing the suffix "a". In the FIG. 5 embodiment, a nerve stimulator needle guidance system 10a includes a cutaneous electrode 12a and a nerve stimulator needle 14a coupled to a nerve stimulator.

The cutaneous electrode 12a includes a nonconductive body 18a, illustrated of generally cylindrical configuration, by way of example only and a lower flange 20a. An electrical lead 26a couples a conductive tip, positioned beneath the flange 20a, to the nerve stimulator. In a manner similar to that with respect to the previous embodiment, the cutaneous electrode 12a includes a bore 24a extending coaxially through a body 18a.

A planar radial slot 66a extends substantially along the entire axial length of the body 18a from the upper end thereof and a plurality of indexing marks 68a appear as a scale along the length of the body adjacent the slot 66a.

A radial indexing arm 70a is fixed to a shaft 27a of the needle 14a and may be employed, in conjunction with the marks 68a, to gauge the depth of penetration of the tip of the needle shaft 27a.

The radial arm 70a may be of sufficient rigidity to be employed for the purpose of advancing the needle shaft 27a into the patient with the anesthesiologist applying a downward force to a portion of the arm 70a which projects radially from the slot 66a.

The technique of employing the nerve stimulator needle guidance system 10a of the alternate embodiment is substantially identical to that employed in conjunction with the previous embodiment, with the exception, however, of enabling the anesthesiologist to utilize the indexing marks as a depth of penetration gauge and also the utilization of the arm 70a for the purpose of advancing the needle shaft into the patient.

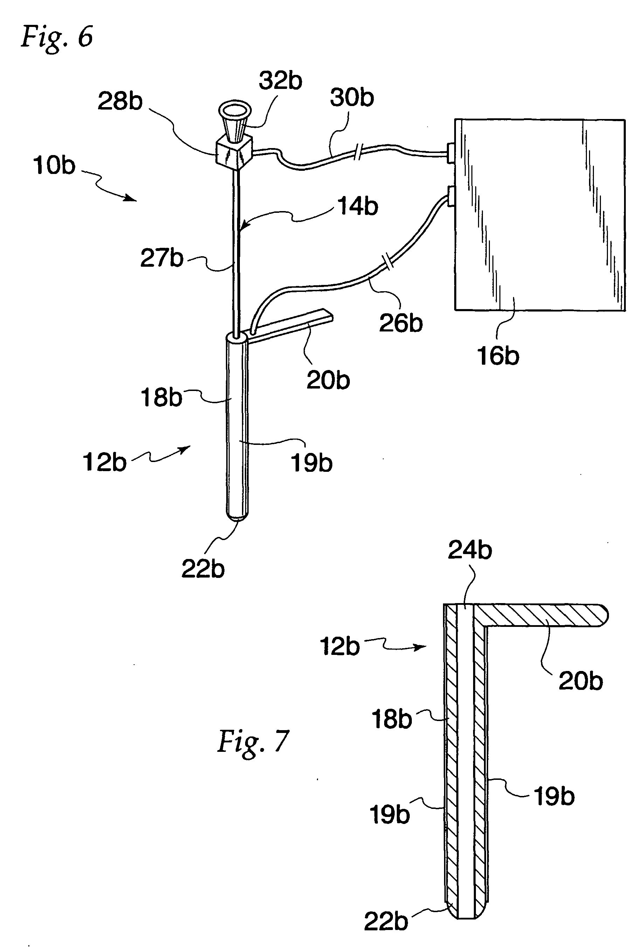

With reference now to FIG. 6 through FIG. 11 wherein a further embodiment of the invention is illustrated, like numerals have been employed to denote like components of the previous embodiments, however, bearing the suffix "b". In this embodiment, a nerve stimulator needle guidance system 10b includes a subcutaneous guiding electrode 12b which is employed to locate a subcutaneous point in registration with a target nerve. A nerve stimulator needle electrode 14b is introduced into the patient through the guiding electrode 12b, with both electrodes being selectively coupled to a nerve stimulator 16b substantially identical to the nerve stimulator 16.

The subcutaneous electrode 12b comprises a hollow, electrically conductive shaft 18b, approximately 16 gauge, i.e. approximately 1.5 mm in diameter. The outer surface of the shaft 18b is coated with a nonconductive layer 19b.

A transverse radial flange 20b projects laterally adjacent the upper end of the shaft 18b and a generally hemispherical electrically conductive blunt tip 22b is positioned at the lower end of the shaft 18b.

Extending coaxially through both the shaft 18b and the conductive tip 22b is a bore 24b, approximately .5 mm in diameter. The tip 22b is electrically coupled to the nerve stimulator 16b through the shaft 18b and an electrical lead 26b.

The nerve stimulator needle 14b is substantially identical to the nerve stimulator needle 14 described in detail with reference to the first embodiment. The outer diameter of the needle shaft 27b is dimensioned to be received within the bore 24b. The needle 14b includes a connector block 28b having an electrical lead 20b to the nerve stimulator 16b and a Luer fitting 32b for coupling through a syringe.

The subcutaneous guiding electrode 12b is well-suited for use in instances wherein a target nerve 52b or nerve plexus is not in close proximity to a patient's skin 56b and as a result, the cutaneous electrode does not provide optimal results.

Instances wherein the subcutaneous guiding electrode 12b may be implemented include the administration of nerve blocks in obese patients, or for example, the administration of a brachial plexus infra clavicular block, a psoas compartment lumbar plexus block, a sciatic nerve block at the popliteal fossa and possibly, in implementing a femoral nerve block.

The nerve stimulator 16b includes a subcutaneous guiding electrode output wherein current flow may be within an overall range of 1 mA through 5 mA, for example, and a nerve stimulator needle output wherein current flow may be within an overall range of .3 to 3.0 mA, for example. By way of further example, minimum subcutaneous guiding electrode current for location of a target nerve may be achieved at 3 mA while minimum stimulator needle current flow may be achieved at .3 mA.

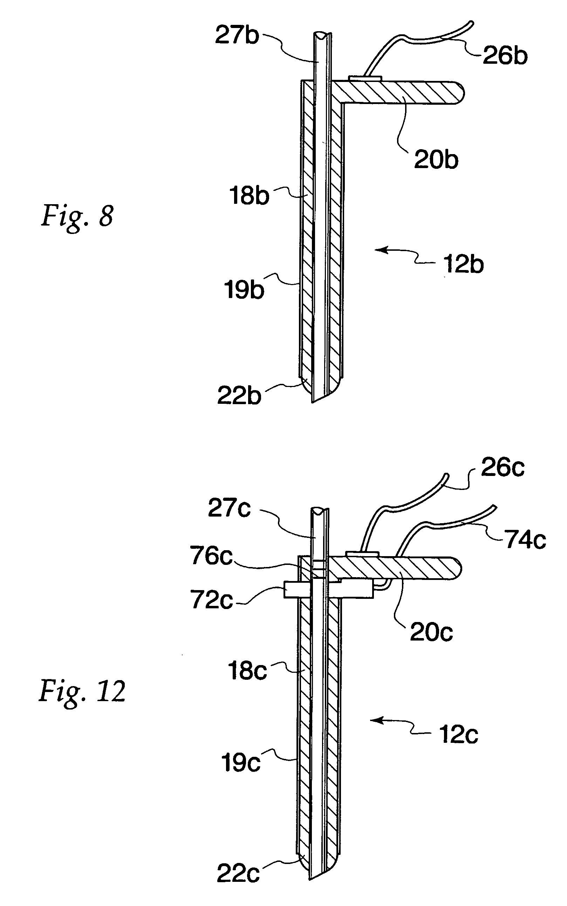

The subcutaneous guiding electrode 12b is introduced through the skin 56b at an approximate location of a target nerve 52b which is selected by anatomic landmarks. An introducer needle, which may comprise the nerve stimulator needle 14b, is carried in the bore 24b and extends just beyond the blunt tip 22b, as illustrated in FIG. 8. The subcutaneous guiding electrode 12b is advanced into the patient until its tip lies at a designated distance beneath the skin 56b, e.g. between 1 and 3 cm.

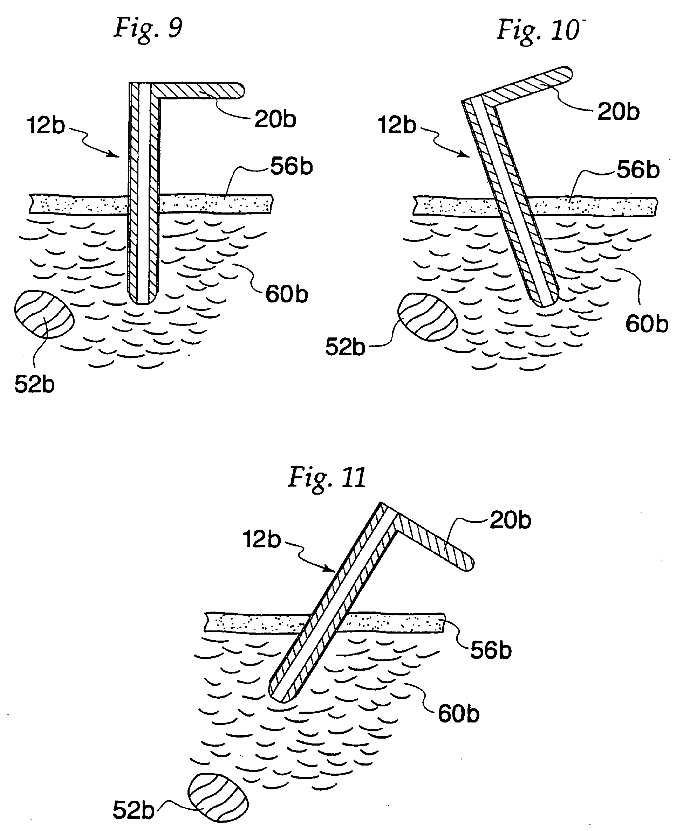

Thereafter, the introducer needle may be completely removed from the bore 24b, as illustrated in FIGS. 9 through 11, or merely retracted partially such that it does not extend beyond the blunt tip 22b.

The nerve stimulator guiding electrode 12b is then manipulated by grasping the radial flange 22b or alternately a collar or other gripping surface adjacent the proximal end of the electrode. Manipulation may proceed in a sweep pattern, pivoting about the point of introduction through the skin 56b as illustrated in FIGS. 9 through 11. During manipulation, nerve stimulation current in the order of 3 mA may be applied.

Due to the relative rigidity of the subcutaneous guiding electrode shaft 18b, as opposed to the nerve stimulator needle shaft 27b, the guiding electrode will indent and deform tissue during the manipulation in search of the target nerve. Because the subcutaneous guiding electrodes surfaces are smooth, negligible or no damage to subcutaneous tissue results from the electrode manipulation in search of the target nerve.

Upon detection of the target nerve through muscle contractions or other neural response, the nerve stimulator current supply to the guiding electrode is switched off. The nerve stimulator needle 14b is either positioned in the bore 24b or, if it has not been removed from the bore 24b, is extended through the tip 22b and current is then applied from the nerve stimulator 16b through the lead 30b to the nerve stimulator needle.

During this procedure, the subcutaneous guiding electrode 12b is held fast and the nerve stimulator needle 14b is advanced toward the target nerve 52b.

When appropriate muscle contractions or other response is elicited at reduced current levels associated with the prescribed distance between the tip of the nerve stimulator needle shaft and the target nerve, the needle is properly positioned.

A further embodiment of the invention is depicted in FIG. 12, in this embodiment, like numerals have been employed to denote like components of the prior embodiments, bearing the suffix "c", however. The FIG. 12 embodiment is substantially the same as the embodiment of FIGS. 6 through 11 except, however, a sensor 72c is provided adjacent the proximal end of a subcutaneous guiding electrode 12c.

The sensor serves to detect when a needle shaft 27c, projects beyond a blunt tip 22c of the subcutaneous guiding electrode 12c and applies a switching signal through an electrical lead 74c. The signal serves to switch the nerve stimulator current supply output from a subcutaneous guiding electrode output to a nerve stimulator needle output.

The sensor 72c may be of conventional configuration and may comprise, for example, an optical sensor which reads indicia 76c imprinted on the needle shaft 27c. Alternate sensors may be utilized.

It should be appreciated that the nerve stimulator needle guidance system of the present invention may be implemented not only for administering an anesthesia blockade but for other medical techniques and practices wherein nerves or a nerve plexus is to be located for treatment such as electromyography, neuro monitoring and therapeutic intervention.

Thus it will be seen that there is provided a nerve stimulator needle guidance system which achieves the various aspects, features and considerations of the present invention and which is well adapted to meet the conditions of practical usage.

As various possible further embodiments might be made of the present invention and various changes might be made in the illustrative embodiments above set forth, without departing from the scope of the invention as defined in the claims, it is to be understood that all matter herein described or shown in the accompanying drawings is to be interpreted as illustrative and not in a limiting sense.

## Claims

1. A nerve stimulator needle guidance system comprising an electrode (12, 12b) for locating a cutaneous or subcutaneous point in proximity with a target nerve or nerve plexus, the electrode having a conductive tip (22, 22b) and a bore (24, 24b) extending through the electrode and the tip, **characterised by** the tip having a smooth surface and a nerve stimulator needle (14, 14b) received within and axially moveable within the bore.

2. A nerve stimulator needle guidance system constructed in accordance with claims 1 further comprising an electrode current supply coupled to the electrode.

3. A needle guidance system as constructed in accordance with claim 2 wherein the electrode current supply comprises a nerve stimulator (16, 16b).

4. A needle guidance system as constructed in accordance with any of claims 1 to 3 further comprising a needle current supply coupled to the needle.

5. A needle guidance system as constructed in accordance with claim 4 wherein the needle current supply comprises a nerve stimulator (16, 16b).

6. A nerve stimulator needle guidance system as constructed in accordance with any of the preceding claims wherein the electrode is a cutaneous electrode (12) in which the tip (22) has said smooth surface for traversing over the surface of a patient's skin while maintaining electrical current flow through the patient.

7. A nerve stimulator needle guidance system as constructed in claim 6 wherein the cutaneous electrode includes an axial slot (66a) extending from the bore; and wherein the needle includes a radial arm (70a) fixed to the needle, the radial arm being received in the slot.

8. A nerve stimulator needle guidance system as constructed in accordance with claim 7 wherein the cutaneous electrode further includes indicia (68a) adjacent the slot, whereby the depth of penetration of the needle may be gauged.

9. A nerve stimulator needle guidance system according to any of claims 6 to 8, wherein the bore extends coaxially through the body of the cutaneous electrode.

10. A nerve stimulator needle guidance system as constructed in accordance with any of claims 6 to 9, wherein the surface of the tip of the cutaneous electrode is curved.

11. A nerve stimulator needle guidance system as constructed in accordance with any of claims 1 to 5, wherein the electrode is a subcutaneous guiding electrode (12b) including a shaft (18b), and wherein the tip has said smooth surface for traversing subcutaneous tissue while maintaining electrical current flow through the patient.

12. A nerve stimulator needle guidance system as constructed in accordance with claim 11 further including a flange (20b), the flange extending laterally from the shaft adjacent a proximal end of the shaft whereby the subcutaneous guiding electrode may be manipulated in a subcutaneous sweep pattern to locate the point in proximity with the target nerve or nerve plexus.

## Patentansprüche

1. Nadelführungssystem für einen Nervenstimulator, welches eine Elektrode (12, 12b) zum Lokalisieren eines kutanen oder subkutanen Punkts in der Nähe eines Zielnervs oder -nervengeflechts aufweist, wobei die Elektrode eine leitfähige Spitze (22, 22b) und eine sich durch die Elektrode und die Spitze erstreckende Bohrung (24, 24b) aufweist, **dadurch gekennzeichnet, daß** die Spitze eine glatte Oberfläche hat und eine Nervenstimulatornadel (14, 14b) innerhalb der Bohrung aufgenommen ist und darin in axialer Richtung bewegt werden kann.

2. Nadelführungssystem für einen Nervenstimulator, aufgebaut gemäß Anspruch 1, welches weiterhin eine mit der Elektrode gekoppelte Elektroden-Stromversorgung aufweist.

3. Nadelführungssystem mit einem Aufbau gemäß Anspruch 2, wobei die Elektroden-Stromversorgung einen Nervenstimulator (16, 16b) aufweist.

4. Nadelführungssystem mit einem Aufbau gemäß einem der Ansprüche 1 bis 3, welches weiterhin eine mit der Nadel gekoppelte Nadel-Stromversorgung aufweist.

5. Nadelführungssystem mit einem Aufbau gemäß Anspruch 4, wobei die Nadel-Stromversorgung einen Nervenstimulator (16, 16b) aufweist.

6. Nadelführungssystem für einen Nervenstimulator, aufgebaut gemäß einem der vorangegangenen Ansprüche, wobei die Elektrode eine kutane Elektrode (12) ist, bei der die Spitze (22) die glatte Oberfläche hat, um die Oberfläche der Haut eines Patienten zu überfahren, während ein elektrischer Stromfluß durch den Patienten aufrechterhalten wird.

7. Nadelführungssystem für einen Nervenstimulator, aufgebaut gemäß Anspruch 6, wobei die kutane Elektrode einen axialen Schlitz (66a) aufweist, der sich von der Bohrung erstreckt, und wobei die Nadel einen an der Nadel befestigten radialen Arm (70a) aufweist und der radiale Arm in dem Schlitz aufgenommen wird.

8. Nadelführungssystem für einen Nervenstimulator, aufgebaut gemäß Anspruch 7, wobei die kutane Elektrode weiterhin Markierungen (68a) neben dem Schlitz aufweist, mittels derer die Penetrationstiefe der Nadel beurteilt bzw. gemessen werden kann.

9. Nadelführungssystem für einen Nervenstimulator nach einem der Ansprüche 6 bis 8, wobei sich die Bohrung koaxial durch den Körper der kutanen Elektrode erstreckt.

10. Nadelführungssystem für einen Nervenstimulator, aufgebaut gemäß einem der Ansprüche 6 bis 9, wobei die Oberfläche der Spitze der kutanen Elektrode gekrümmt bzw. bogenförmig ist.

11. Nadelführungssystem für einen Nervenstimulator, aufgebaut gemäß einem der Ansprüche 1 bis 5, wobei die Elektrode eine subkutane Führungselektrode (12b) ist, die einen Schaft (18b) aufweist, und wobei die Spitze die glatte Oberfläche hat, um subkutanes Gewebe zu überfahren, während ein elektrischer Stromfluß durch den Patienten aufrechterhalten wird.

12. Nadelführungssystem für einen Nervenstimulator, aufgebaut gemäß Anspruch 11, welches weiterhin einen Flansch (20b) aufweist, wobei sich der Flansch lateral von dem Schaft neben ein proximales Ende des Schafts erstreckt, wodurch die subkutane Führungselektrode in einem subkutanen Abtastmuster bewegt bzw. manipuliert werden kann, um den Punkt in der Nähe des Zielnervs oder -nervengeflechts zu lokalisieren.

## Revendications

1. Système de guidage d'aiguille neurostimulatrice comprenant une électrode (12, 12b) pour localiser un point cutané ou sous-cutané à proximité d'un nerf cible ou plexus nerveux cible, l'électrode ayant une pointe conductrice (22, 22b) et un alésage (24, 24b) s'étendant à travers l'électrode et la pointe, **caractérisé par** la pointe qui a une surface lisse et une aiguille neurostimulatrice (14, 14b) reçue à l'intérieur de l'alésage et axialement mobile à l'intérieur de celui-ci.

2. Système de guidage d'aiguille neurostimulatrice selon la revendication 1, comprenant en outre une alimentation de courant d'électrode couplée à l'électrode.

3. Système de guidage d'aiguille selon la revendication 2, dans lequel l'alimentation de courant d'électrode comprend un neurostimulateur (16, 16b).

4. Système de guidage d'aiguille selon l'une quelconque des revendications 1 à 3, comprenant en outre une alimentation de courant d'aiguille couplée à l'aiguille.

5. Système de guidage d'aiguille selon la revendication 4, dans lequel l'alimentation de courant d'aiguille comprend un neurostimulateur (16, 16b).

6. Système de guidage d'aiguille neurostimulatrice selon l'une quelconque des revendications précédentes, dans lequel l'électrode est une électrode cutanée (12) dans laquelle la pointe (22) a ladite surface lisse pour traverser la surface de la peau d'un patient tout en maintenant la circulation du courant électrique à travers le patient.

7. Système de guidage d'aiguille neurostimulatrice selon la revendication 6, dans lequel l'électrode cutanée comprend une fente axiale (66a) s'étendant à partir de l'alésage ; et dans lequel l'aiguille comprend un bras radial (70a) fixé sur l'aiguille, le bras radial étant reçu dans la fente.

8. Système de guidage d'aiguille neurostimulatrice selon la revendication 7, dans lequel l'électrode cutanée comprend en outre un indice (68a) adjacent à la fente, moyennant quoi la profondeur de pénétration de l'aiguille peut être mesurée.

9. Système de guidage d'aiguille neurostimulatrice selon l'une quelconque des revendications 6 à 8, dans lequel l'alésage s'étend de manière coaxiale à travers le corps de l'électrode cutanée.

10. Système de guidage d'aiguille neurostimulatrice selon l'une quelconque des revendications 6 à 9, dans lequel la surface de la pointe de l'électrode cutanée est incurvée.

11. Système de guidage d'aiguille neurostimulatrice selon l'une quelconque des revendications 1 à 5, dans lequel l'électrode est une électrode de guidage sous-cutanée (2b) comprenant une tige (18b) et dans lequel la pointe a ladite surface lisse pour traverser le tissu sous-cutané tout en maintenant la circulation du courant électrique à travers le patient.

12. Système de guidage d'aiguille neurostimulatrice selon la revendication 11, comprenant en outre un rebord (20b), le rebord s'étendant latéralement à partir de la tige, de manière adjacente à une extrémité de la tige, moyennant quoi l'électrode de guidage sous-cutanée peut être manipulée selon un modèle de balayage sous-cutané pour localiser le point à proximité du nerf cible ou du plexus nerveux cible.
